(19) Europäisches Patentamt
European Patent Office
Office européen des brevets

(11) Veröffentlichungsnummer: **0 010 616**
**B1**

(12) **EUROPÄISCHE PATENTSCHRIFT**

(45) Veröffentlichungstag der Patentschrift: **29.04.81**

(51) Int. Cl.³: **A 01 N 41/12** //C07C145/02

(21) Anmeldenummer: **79103662.7**

(22) Anmeldetag: **27.09.79**

(54) **4-Nitro-2-trichlormethylphenylsulfenamide und diese enthaltende Fungizide, deren Herstellung und Verwendung bei der Bekämpfung von Pilzen.**

(30) Priorität: **06.10.78 DE 2843644**

(43) Veröffentlichungstag der Anmeldung:
**14.05.80 Patentblatt 80/10**

(45) Bekanntmachung des Hinweises auf die Patenterteilung:
**29.04.81 Patentblatt 81/17**

(84) Benannte Vertragsstaaten:
**AT BE CH DE FR GB IT LU NL SE**

(56) Entgegenhaltungen:
**CHEMICAL ABSTRACTS, Vol. 61, Nr. 5, 31. August 1964, Spalte 5550d, Columbus, Ohio, USA,**
**A. BARUFFINI et al.: "Arylsulfenamides with antifungal activity"**

(73) Patentinhaber: **BASF Aktiengesellschaft**
**Carl-Bosch-Strasse 38**
**D-6700 Ludwigshafen (DE)**

(72) Erfinder: **Hagen, Helmut, Dr. Dipl.-Ing.**
**Max-Slevogt-Strasse 17E**
**D-6710 Frankenthal (DE)**
Erfinder: **Pommer, Ernst-Heinrich, Dr.**
**Berliner Platz 7**
**D-6703 Limburgerhof (DE)**
Erfinder: **Reuther, Wolfgang, Dr. Dipl.-Chem.**
**Am Pferchelhang 16**
**D-6900 Heidelberg-Ziegelhausen (DE)**
Erfinder: **Ziegler, Hans, Dr. Dipl.-Chem.**
**Hans-Purrmann-Strasse 4**
**D-6710 Frankenthal (DE)**

**EP 0 010 616 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 4-Nitro-2-trichlormethylphenylsulfenamide und diese enthaltende Fungizide, deren Herstellung und Verwendung bei der Bekämpfung von Pilzen

Die vorliegende Erfindung betrifft neue, wertvolle 4-Nitro-2-trichlormethylphenylsulfenamide mit fungizider und bakterizider Wirkung, Verfahren zu ihrer Herstellung, Fungizide, die diese Verbindungen als Wirkstoffe enthalten, und Verfahren zur Bekämpfung von Pilzen mit diesen Verbindungen.

Es ist bekannt, N-Trichlormethylthiophthalimid oder N-Trichlormethylthiotetrahydrophthalimid (Chemical Week 1972, June 21, S. 46 und 63) als Fungizide zu verwenden. Es ist ferner bekannt, 4-Nitrophenylsulfenamide als Fungizide zu verwenden (C.A. *61*, 5550 d (1964)). Ihre Wirkung ist jedoch unbefriedigend.

Aufgabe der vorliegenden Erfindung ist die Entwicklung neuer Wirkstoffe mit besserer fungizider Wirkung.

Es wurde gefunden, daß 4-Nitro-2-trichlormethylphenylsulfenamide der Formel

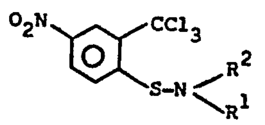

in der $R^1$ und $R^2$ gleich oder voneinander verschieden sind und Wasserstoff, einen gegebenenfalls durch Alkoxy, Hydroxy, Trialkoxysilyl substituierten Alkylrest mit 1 bis 18 Kohlenstoffatomen einen Alkenylrest mit 3 bis 5 Kohlenstoffatomen, Cycloalkylrest mit 5 bis 8 Kohlenstoffatomen, einen gegebenenfalls durch Halogen substituierten Aralkylrest mit 7 bis 9 Kohlenstoffatomen, einen gegebenenfalls durch Alkyl, Halogen, Nitro, Halogenalkyl substituierten Phenylrest bedeuten, $R^1$ ferner einen 1 bis 2 Heteroatome enthaltenden 5- bis 6-gliedrigen ungesättigten Methylenring mit Stickstoff oder Schwefel als Heteroatomen oder dessen Benzhomologe bedeuten oder $R^1$ und $R^2$ zusammen mit dem Stickstoffatom, dessen Substituenten sie sind, einen 5- bis 7-gliedrigen gegebenenfalls ungesättigten Methylenring bedeuten, der gegebenenfalls durch Alkyl, Phenyl, Nitro substituiert ist und gegebenenfalls im Methylenring noch Stickstoff oder Sauerstoff enthalten kann und $R^1$ ferner den Rest 4-Nitro-2-trichlormethylphenylsulfenyl bedeutet.

$R^1$ und $R^2$ bedeuten beispielsweise geradkettige oder verzweigte Alkylreste mit 1 bis 18 C-Atomen, gegebenenfalls substituiert durch Hydroxyl-, Trialkoxysilyl- oder Alkoxygruppen mit 1 bis 3 C-Atomen im Alkyl, z.B. Methyl, Äthyl, iso-Propyl, n-Propyl, iso-Butyl, Octyl, Stearyl, n-Dodecyl, Methoxyäthyl, Hydroxyäthyl, Triäthoxysilylpropyl oder sie bedeuten beispielsweise Cycloalkylreste mit 5 bis 8 C-Atomen, z.B. Cyclohexyl;

oder sie bedeuten beispielsweise geradkettige oder verzweigte Alkenyle mit 3 bis 5 C-Atomen, z.B. Allyl, Methallyl;

oder sie bedeuten einen Aralkylrest mit 7 bis 9 C-Atomen beispielsweise Benzyl oder Phenyläthyl, die am Phenyl gegebenenfalls 1- bis 3-fach zum Beispiel durch Halogenatome substituiert sein können, z.B. Chlorbenzyl, Dichlorbenzyl.

oder sie bedeuten einen Phenylrest z.B. einen gegebenenfalls 1- bis 3-fach substituierten Phenylrest, der durch Halogenatome, insbesondere Chloratome, oder Nitrogruppen, oder Trifluormethylgruppen substituiert sein kann, z.B. Dichlorphenyl, Nitrophenyl, Chlor-Nitrophenyl oder Chlortrifluormethylphenyl.

$R^1$ kann ferner einen heterocyclischen Rest bedeuten, z.B. den Pyridyl-, Benzthiazolyl- oder Thiazolylrest, wobei dann $R^2$ Wasserstoff oder Alkyl bedeuten kann.

Als heterocyclische Reste kommen für $R^1 + R^2$ zusammen mit N beispielsweise gesättigte oder ungesättigte Heterocyclen in Betracht, die gegebenenfalls außer dem N Sitckstoff- oder Sauerstoffatome enthalten können, z.B. der Rest von Morpholin, 3,5-Dimethylmorpholin, Pyrrolidin, Pyrrol, Piperidin, Hexahydro-Azepin oder Imidazol, die durch Methyl, Äthyl, Isopropyl, Phenyl, Nitro substituiert sein können.

Von den für $R^1$ und $R^2$ genannten Bedeutungen werden bevorzugt: Wasserstoff, Alkyl von 1 bis 12 C-Atomen, gegebenenfalls substituiert durch Hydroxyl; ferner Alkenyl, Phenyl, Benzyl gegebenenfalls substituiert durch Chlor; ferner Thiazol, Benzthiazol; ferner Pyridin;

für $R^1$ und $R^2$ zusammen mit N werden bevorzugt: Imidazol, gegebenenfalls durch Alkyl oder Phenyl substituiert; ferner Morpholin gegebenenfalls mehrfach substituiert durch Alkyl.

Die folgende Aufstellung zeigt beispielsweise bevorzugte Kombinationen von $R^1$ und $R^2$.

| $R^1$ | $R^2$ |
|---|---|
| H | H |
| Äthyl | Äthyl |
| iso-Propyl | H |
| n-Propyl | H |
| n-Octyl | H |
| Cyclohexyl | H |
| Cyclohexyl | Methyl |
| p-Nitrophenyl | H |
| Benzyl | H |
| 2-Thiazolyl | H |
| Allyl | H |
| 2-Hydroxyäthyl | H |
| 2-Pyridinyl | H |
| 2-Chlorbenzyl | H |
| 2-Benzothiazol | H |

Für $R^1$ und $R^2$ zusammen mit N werden beispielsweise als bevorzugt genannt.

Imidazol-,
Imidazol-2-Phenyl-,
Imidazol-2-Methyl-,
Imidazol-2-Isopropyl,
Imidazol-4-Nitro,
Imidazol-5-Nitro,
3,5-Dimethylmorpholin.

Die Herstellung der neuen Verbindungen kann durch Umsetzung von 4-Nitro-2-trichlormethylbenzolsulfensäurechlorid mit einer eine Aminogruppe enthaltenden Verbindung, bei der $R^1$ und $R^2$ die oben angegebenen Bedeutungen haben, in einem inerten Lösungsmittel bei Temperaturen von –40°C bis 120°C in Gegenwart eines chlorwasserstoffbindenden Mittels erfolgen.

Die Umsetzung wird durch folgende Reaktionsgleichung beschrieben.

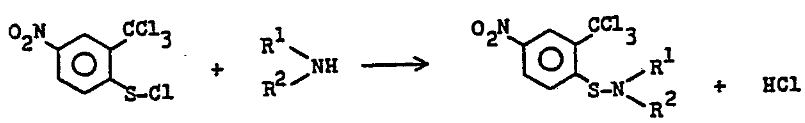

Für die Umsetzungen kommen als inerte Lösungsmittel beispielsweise gesättigte aliphatische oder cyclische Äther, z.B. Diäthyläther, Diisopropyläther, Tetrahydrofuran oder Dioxan, ferner wasserfreie Essigsäure in Frage, ferner aliphatische Carbonsäureester z.B. Essigsäureäthylester; ferner halogenierte Kohlenwasserstoffe, z.B. Chloroform oder Methylenchlorid. Zum Binden des freiwerdenden Chlorwasserstoffs können beispielsweise ein Überschuß des Amins der Formel $HNR^1R^2$ oder vorzugsweise Basen wie tertiäre Amine z.B. Triäthylamin, Pyridin, ferner Natrium- oder Kaliumacetat oder Alkalicarbonate, z.B. Natriumcarbonat verwendet werden.

Der für die Umsetzung bevorzugte Temperaturbereich liegt bei Temperaturen von –10°C bis 60°C.

Das als Ausgangsprodukt verwendete 4-Nitro-2-trichlormethylbenzolsulfensäurechlorid kann beispielsweise durch Chlorierung von 5-Nitrobenzo-1,2-dithio-3-thion in einem inerten Lösungsmittel bei Temperaturen zwischen –20°C und 100°C, vorzugsweise zwischen 0°C und 50°C hergestellt werden. Als inerte Lösungsmittel kommen z.B. chlorierte Kohlenwasserstoffe wie Tetrachlorkohlenstoff, Chloroform, Dichloräthan, usf. in Betracht.

Die Umsetzung läßt sich durch folgende Reaktionsgleichung beschrieben:

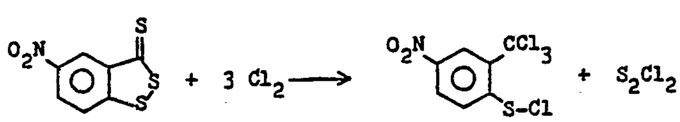

Das als Ausgangsverbindung verwendete 5-Nitrobenzo-1,2-dithio-3-thion kann nach dem in der DE-A-24 60 783 beschriebenen Verfahren bequem hergestellt werden.

Die folgende Vorschrift erläutert dieses Verfahren.

51,5 g 2-Chlor-5-nitrobenzylchlorid wurden mit 24 g Schwefel in 500 ml Methanol 30 Minuten auf 50°C erwärmt. Dann setzt man innerhalb einer Stunde 50,5 g Triäthylamin zu und erhitzt das Reaktionsgemisch 20 Stunden lang auf 65°C. Nach dem Abkühlen wird der ausgefallene Feststoff abgesaugt und mit Wasser gewaschen.

Man erhält 50,5 g 5-Nitrobenz-1,2-dithio-3-thion, entsprechend 89% der Theorie. Der Schmelzpunkt beträgt 172 bis 173°C (Zersetzung).

Herstellung der Verbindung

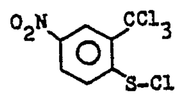

115 g 5-Nitrobenzo-1,2-dithio-3-thion werden in 1000 ml Tetrachlorkohlenstoff dispergiert und bei 10°C 150 g Chlor eingeleitet. Das Reaktionsgemisch wird noch 12 Stunden bei Raumtemperatur (25°C) gerührt. Das Lösungsmittel und Schwefelchlorid werden danach im Vakuum einer Wasserstrahlpumpe abdestilliert. Der Rückstand wird mit 400 ml Diäthyläther versetzt und filtriert. Die Ätherlösung wird eingeengt und im Vakuum destilliert. Bei $Kp_1 = 156°C$ bis 160°C werden 128 g (83% der Theorie) 2-Trichlormethyl-4-nitro-benzolsulfensäurechlorid erhalten. Die Verbindung hat einen Schmelzpunkt von 56°C bis 57°C (aus Ligroin).

Die Herstellung der neuen Verbindungen wird in den folgenden Beispielen erläutert.

Beispiel 1

4-Nitro-2-trichlormethylmethylphenylsulfenamid

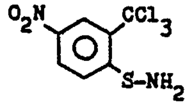

In einer Rührapparatur werden 200 ml 20%-ige (Gew.%) wäßrige $NH_3$-Lösung vorgelegt und langsam unter Rühren bei 20°C mit 61,4 g 4-Nitro-2-trichlormethylbenzolsulfensäurechlorid, gelöst in 200 ml Diäthyläther, versetzt. Nach einstündigem Rühren bei 20°C wird die wäßrige Phase abgetrennt, die Ätherphase mit Wasser gewaschen und der Äther verdampft.

Aus Toluol erhält man 45 g 4-Nitro-2-trichlormethylphenylsulfenamid mit Fp = 105°C.

Ausbeute 70% der Theorie

| Analyse: | | C | H | N | S | Cl | O |
|---|---|---|---|---|---|---|---|
| | gef. | 29,2 | 1,7 | 9,8 | 11,3 | 36,9 | 11,1 |
| | ber. | 29,2 | 1,7 | 9,7 | 11,2 | 37,0 | 11,2 |

Beispiel 2

N-Methyl-4-nitro-2-trichlormethylphenylsulfenamid

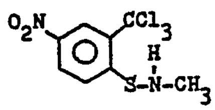

In eine Lösung von 61,4 g 4-Nitro-2-trichlormethylbenzolsulfensäurechlorid in 200 ml Diäthyläther wird bei 0°C 10 l Methylamin eingeleitet. Nach 6 Stunden Rühren bei 25°C wird auf Eiswasser gegossen, die wäßrige Phase abgetrennt und zur Trockene eingedampft. Man erhält 51 g N-Methyl-4-nitro-2-trichlormethylphenylsulfenamid als Öl. ($n_D^{20} = 1,6610$)

Ausbeute: 85% der Theorie

| Analyse: | | C | H | N | S | Cl | O |
|---|---|---|---|---|---|---|---|
| | gef. | 31,9 | 2,4 | 9,1 | 10,5 | 35,4 | 10,7 |
| | ber. | 31,8 | 2,3 | 9,3 | 10,6 | 35,3 | 10,6 |

Beispiel 3.

N-Äthyl-4-nitro-2-trichlormethylphenylsulfenamid

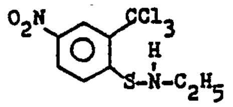

61,4 g 4-Nitro-2-trichlormethylbenzolsulfensäurechlorid werden analog Beispiel 1 mit wäßriger Äthylaminlösung umgesetzt.

Man erhält 56 g N-Äthyl-4-nitro-2-trichlormethylphenylsulfenamid vom Fp. 80°.

Ausbeute: 90% der Theorie

| Analyse: | | C | H | N | S | Cl | O |
|---|---|---|---|---|---|---|---|
| | gef. | 34,2 | 3,1 | 9,0 | 9,9 | 33,7 | 10,2 |
| | ber. | 34,2 | 2,9 | 8,9 | 10,1 | 33,8 | 10,1 |

Beispiel 4.

N,N-Diisopropyl-4-nitro-2-trichlormethylphenylsulfenamid

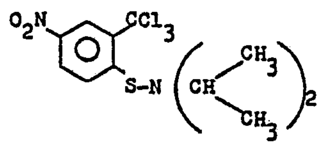

In einer Rührapparatur werden 61,4 g 4-Nitro-2-trichlormethylbenzolsulfensäurechlorid in 200 ml Äther zu 40 g Diisopropylamin, in 400 ml Äther gelöst, bei 10°C zugetropft. Nach vierstündiger Reaktion bei 25°C wird abgesaugt und eingeengt. Man erhält 70 g N,N-Diisopropyl-4-nitro-2-trichlormethylphenylsulfenamid vom Fp: 104°C.

Ausbeute: 95% der Theorie

| Analyse: | | C | H | N | S | Cl | O |
|---|---|---|---|---|---|---|---|
| | gef. | 41,9 | 4,6 | 7,6 | 8,8 | 28,7 | 8,4 |
| | ber. | 42,0 | 4,6 | 7,5 | 8,6 | 28,7 | 9,6 |

Beispiel 5.

N,N-Di-(2-Methoxyäthyl)-4-nitro-2-trichlorphenylsulfenamid

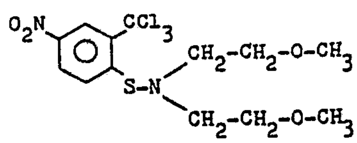

Analog Beispiel 4 wird 4-Nitro-2-trichlormethylbenzolsulfensäurechlorid mit Di(2-Methoxyäthyl)amin umgesetzt.

Man erhält 77 g N,N-Di-(2-Methoxyäthyl)-4-nitro-2-trichlormethylphenylsulfenamid als helles Öl. ($n_D^{20} = 1{,}6018$)

Ausbeute: 95% der Theorie

| Analyse: | | C | H | N | S | Cl | O |
|---|---|---|---|---|---|---|---|
| | gef. | 38,5 | 4,2 | 6,9 | 8,0 | 26,6 | 15,9 |
| | ber. | 38,7 | 4,2 | 6,9 | 7,9 | 26,4 | 15,9 |

Beispiel 6.

N-(2-Hydroxyäthyl)-4-nitro-2-trichlormethylphenylsulfenamid

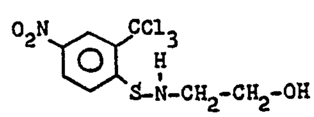

61,4 g 4-Nitro-2-trichlormethylbenzolsulfensäurechlorid werden analog Beispiel 4 mit 24 g 2-Aminoäthanol umgesetzt.

Man erhält 60 g N-(2-Hydroxyäthyl)-4-nitro-2-trichlormethylphenylsulfenamid vom Fp. 85°C

Ausbeute: 90% der Theorie

| Analyse: | | C | H | N | S | Cl | O |
|---|---|---|---|---|---|---|---|
| | gef. | 32,4 | 2,5 | 8,3 | 9,9 | 32,2 | 14,6 |
| | ber. | 32,6 | 2,7 | 8,4 | 9,7 | 32,1 | 14,5 |

Beispiel 7.

N-Allyl-4-nitro-2-trichlormethylphenylsulfenamid

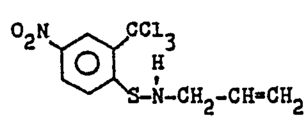

61,4 g 4-Nitro-2-trichlormethylbenzolsulfensäurechlorid werden analog Beispiel 4 mit 23 g Allylamin umgesetzt. Das Hydrochlorid wird abgesaugt und die Ätherphase eingeengt. Man erhält 62 g N-Allyl-4-nitro-2-trichlormethylphenylsulfenamid als helles Öl.

Ausbeute: 95% der Theorie

| Analyse: | C | H | N | S | Cl | O |
|---|---|---|---|---|---|---|
| gef. | 36,6 | 2,9 | 8,6 | 9,9 | 32,3 | 9,7 |
| ber. | 36,6 | 2,7 | 8,5 | 9,8 | 32,5 | 9,8 |

Beispiel 8.

(1,3-Thiazol-2-yl)-4-nitro-2-trichlormethylphenylsulfenamid

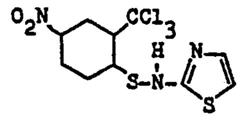

40 g 2-Aminothiazol werden in 500 ml Tetrahydrofuran gelöst und unter Rühren bei 10°C mit 61,4 g 4-Nitro-2-trichlormethylbenzolsulfensäurechlorid versetzt. Durch Zugabe von Wasser wird das Sulfenamid ausgefällt, abgesaugt und aus Essigester umkristallisiert. Man erhält 50 g N-(1,3-Thiazol-2-yl)-4-nitro-2-trichlormethylphenylsulfenamid vom Fp.: 165°C

Ausbeute: 70% der Theorie

| Analyse: | C | H | N | S | Cl | O |
|---|---|---|---|---|---|---|
| gef. | 32,3 | 1,7 | 11,3 | 17,4 | 28,7 | 8,6 |
| ber. | 32,4 | 1,6 | 11,3 | 17,3 | 28,7 | 8,6 |

Beispiel 9.

(2-Isopropylimidazol-1-yl)-4-nitro-2-trichlormethylthiobenzol

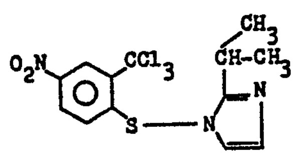

61,4 g 4-Nitro-2-trichlormethylbenzolsulfensäurechlorid werden mit 44 g 2-Isopropylimidazol in Äther umgesetzt. Nach Entfernen des Lösungsmittels und Umkristallisation aus Äther erhält man 53 g (2-Isopropylimidazol-1-yl)-4-nitro-2-trichlormethylthiobenzol vom Fp. 135°C als gelbe Kristalle.

Ausbeute: 72% der Theorie

| Analyse: | C | H | N | S | Cl | O |
|---|---|---|---|---|---|---|
| gef. | 41,4 | 3,2 | 11,1 | 8,2 | 27,9 | 8,4 |
| ber. | 40,9 | 3,1 | 11,0 | 8,4 | 28,0 | 8,4 |

Beispiel 10.

N,N-Diäthyl-(4-nitro-2-trichlormethylphenyl)-sulfenamid

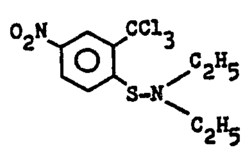

Darstellung aus 4-Nitro-2-trichlormethylbenzolsulfensäurechlorid und Diäthylamin erfolgt analog Beispiel 4.

Man erhält 65 g N,N-Diäthyl-(4-nitro-2-trichlormethylphenyl)-sulfenamid vom Fp. 60°C.

Ausbeute: 95% der Theorie

| Analyse: | C | H | N | S | Cl | O |
|---|---|---|---|---|---|---|
| gef. | 38,3 | 3,7 | 8,4 | 9,3 | 31,1 | 9,2 |
| ber. | 38,4 | 3,8 | 8,2 | 9,3 | 31,0 | 9,3 |

In entsprechender Weise werden die in den folgenden Beispielen beschriebenen Verbindungen erhalten.

Beispiel 11.
(Morpholin-1-yl)-4-nitro-2-trichlormethylthiobenzol

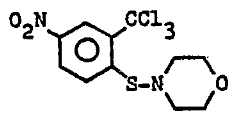

Fp.: 122°C

Beispiel 12.
N,N-Dimethyl-4-nitro-2-trichlormethylphenylsulfenamid

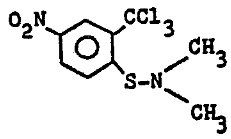

Öl

Beispiel 13.
N-(2-Propyl-4-nitro-2-trichlormethylphenylsulfenamid

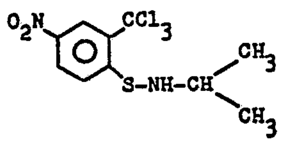

Öl

Beispiel 14.
N-(2-Methylpropyl-(1))-4-nitro-2-trichlormethylphenylsulfenamid

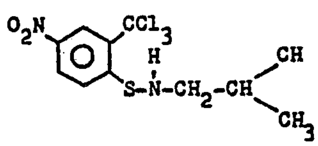

Öl

Beispiel 15.
N-(Octyl-(1)-)-4-nitro-2-trichlormethylphenylsulfenamid

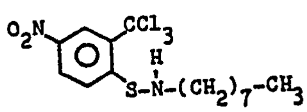

Öl

Beispiel 16.
N-(n-Propyl-(1)-)-4-nitro-2-trichlormethylphenylsulfenamid

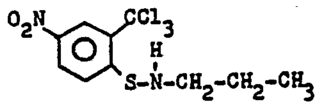

Öl

Beispiel 17.
N,N-(Diallyl)-4-nitro-2-trichlormethylphenylsulfenamid

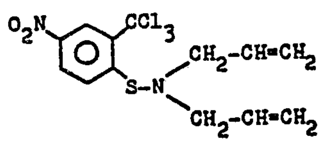

Öl

Beispiel 18.
N-(Benzyl)-4-nitro-2-trichlormethylphenylsulfenamid

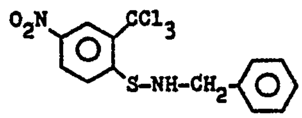

Öl

Beispiel 19.
N-Methyl-N-benzyl-4-nitro-2-trichlormethylphenylsulfenamid

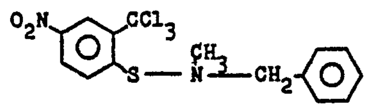

Öl

Beispiel 20.
N-(4-Chlorbenzyl)-4-nitro-2-trichlormethylphenylsulfenamid

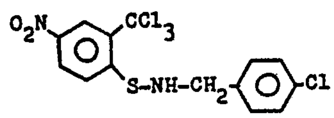

Öl

Beispiel 21.
N-(2-Chlorbenzyl)-4-nitro-2-trichlormethylphenylsulfenamid

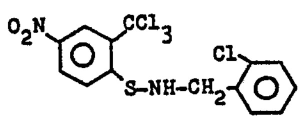

Öl

Beispiel 22.
N-Äthyl-N-methallyl-(4-nitro-2-trichlormethylphenyl)-sulfenamid

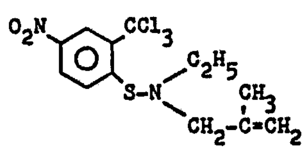

Öl

Beispiel 23.
N-Stearyl-(4-nitro-2-trichlormethylphenyl)-sulfenamid

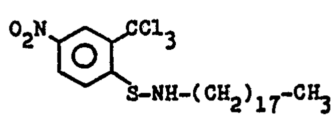

Öl

Beispiel 24.
N-(2-Hydroxyäthyl-(1)-)-4-nitro-2-trichlormethylphenylsulfenamid

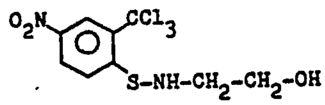

Fp.: 85°C

Beispiel 25.
N-Cyclohexyl-(4-nitro-2-trichlormethylphenyl)-sulfenamid

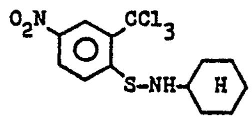

Fp.: 77°C

Beispiel 26.
N-Phenyl-(4-nitro-2-trichlormethylphenyl)-sulfenamid

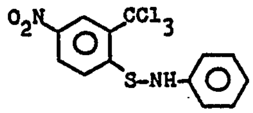

Fp.: 139°C

Beispiel 27.
N-(4-Toluyl)-4-nitro-2-trichlormethylphenylsulfenamid

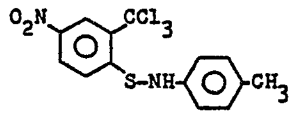

Fp.: 136°C

Beispiel 28.
N-(2,5-Dichlorphenyl)-4-nitro-2-trichlormethylphenylsulfenamid

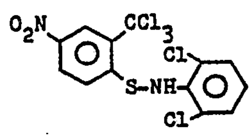

Fp.: 139°C

Beispiel 29.
N-(3,5-Dichlorphenyl)-4-nitro-2-trichlormethylphenylsulfenamid

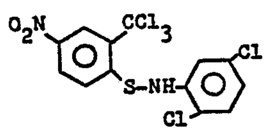

Öl

Beispiel 30.
N-(3,4-Dichlorphenyl)-4-nitro-2-trichlormethylphenylsulfenamid

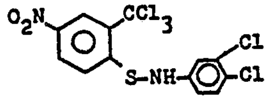

Öl

Beispiel 31.
N-(4-Nitrophenyl)-4-nitro-2-trichlormethylphenylsulfenamid

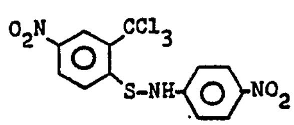

Öl

Beispiel 32.
N-Phenyl-bis-(4-nitro-2-trichlormethylphenyl)-sulfenylamin

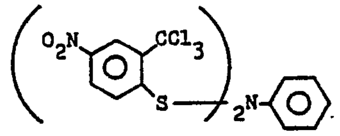

Öl

Beispiel 33.
(Pyrrolidin-1-yl)-4-nitro-2-trichlormethylthiobenzol

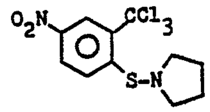

Fp.: 130°C

Beispiel 34.
(Piperidin-1-yl)-4-nitro-2-trichlormethylthiobenzol

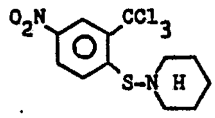

Fp.: 112°C

Beispiel 35.
(3,5-Dimethylmorpholin-1-yl)-4-nitro-2-trichlormethylthiobenzol

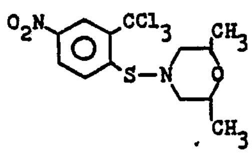

Fp.: 87°C

Beispiel 36.
(Hexahydro-Azepin-1-yl)-4-nitro-2-trichlormethylthiobenzol

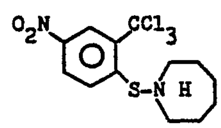

Fp.: 90°C

Beispiel 37.
N-(Pyridin-2-yl)-4-nitro-2-trichlormethylphenylsulfenamid

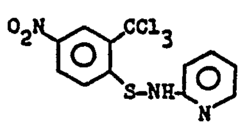

Fp.: 200°C

Beispiel 38.
(2-Aethylimidazol-1-yl)-4-nitro-2-trichlormethylthiobenzol

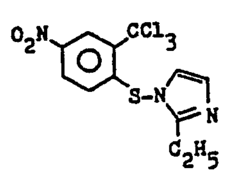

Fp.: 112°C

Beispiel 39.
(2-Methylimidazol-1-yl)-4-nitro-2-trichlormethylthiobenzol

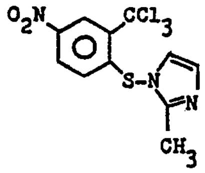

Fp.: 90°C

Beispiel 40.
(2-Phenylimidazol-1-yl)-4-nitro-2-trichlormethylthiobenzol

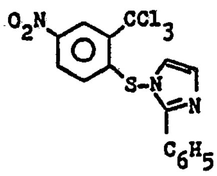

Fp.: 164°C

Beispiel 41.
N-Äthyl-N-(3-chlorphenyl)-4-nitro-2-trichlormethylphenylsulfenamid

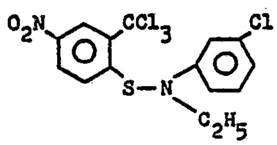

Öl

Beispiel 42.
N-(1,3-Benzthiazol-2-yl)-4-nitro-2-trichlormethylphenylsulfenamid

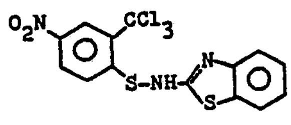

Fp.: 240°C

Beispiel 43.
N-(3-Trifluormethylphenyl)-4-nitro-2-trichlormethylphenylsulfenamid

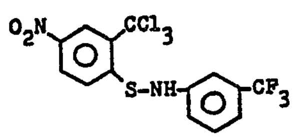

Fp.: 120°C

Beispiel 44.
N-(5-Trifluormethyl-2-chlorphenyl)-4-nitro-2-trichlormethylphenylsulfenamid

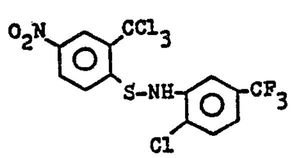

Fp.: 112°C

Beispiel 45.
(Pyrrol-1-yl)-4-nitro-2-trichlormethylthiobenzol

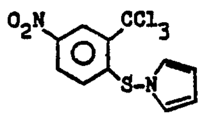

Fp.: 155°C

Beispiel 46.
N-(3-Triäthoxysilyl-propyl)-4-nitro-2-trichlormethylphenylsulfenamid

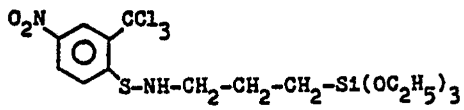

Öl

Beispiel 47.
N-Cyclohexyl-N-methyl-4-nitro-2-trichlormethylphenylsulfenamid

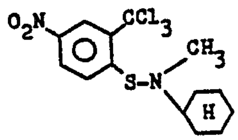

Öl

Beispiel 48.
(Imidazol-1-yl)-4-nitro-2-trichlormethylthiobenzol

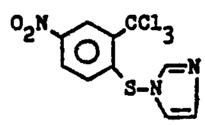

Öl

Beispiel 49.
(1,2-Benzthiazol-3-yl)-4-nitro-2-trichlormethylphenylsulfenamid

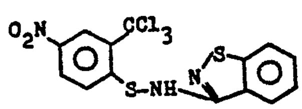

Öl

Beispiel 50.
[4(5)-Nitroimidazol-1-yl]-4-nitro-2-trichlormethylthiosenol

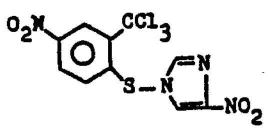

Fp.: 199°C

Die neuen Verbindungen besitzen eine sehr gute fungitoxische Wirksamkeit gegenüber verschiedenen Arten von Schadpilzen, die im Pflanzenschutz oder Materialschutz wirtschaftlich bedeutend sind. Das betrifft insbesondere phytopathogene Pilze aus der Klasse der Phycomyceten, beispielsweise Plasmopara viticola an Reben, Pseudoperonospora humuli an Hopfen, Septoria nodorum an Weizen und Peronospora tabacina an Tabak. Aus anderen Pilzklassen sind folgende materialschädigende Pilze anzuführen: Aspergillus niger, Penicillium glaucum, Chaetomium globosum sowie Coniophora puteana und Serpula lacrimans. Die fungiziden Mittel enthalten 0,1 bis 95% (Gew.%) Wirkstoff, vorzugsweise 0,5 bis 90%. Die Aufwandmengen liegen je nach Art des gewünschten Effektes zwischen 0,001 und 3 kg oder mehr, vorzugsweise jedoch zwischen 0,01 und 1 kg Wirkstoff/ha. Bei der Anwendung der Wirkstoffe im Materialschutz, z.B. als Fungizide für Anstrichfarben, betragen die Aufwandmengen 0,5—5% Wirkstoff, bezogen auf das Gesamtgewicht der zu konservierenden Stoffe, beispielsweise Farben. Die neuen Wirkstoffe können auch als fungizid wirksame Bestandteile öliger Holzschutzmittel zum Schutz von Holz gegen holzzerstörende oder holzverfärbende Pilze eingesetzt werden. Die Anwendung erfolgte in der Weise, daß man das Holz mit diesen Mitteln behandelt, beispielsweise tränkt oder anstreicht.

Einige der neuen Substanzen zeigen auch eine gute bakterizide Wirksamkeit.

Die Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatischen Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und evtl. Lösungsmittel oder Öl bestehende Konzentrate hergestellt werden, die zur Verdünnung mit Wasser geeignet sind.

An oberflächenaktiven Stoffen sind zu nennen:

Alkali-, Erdalkali-, Ammoniumsalze von Ligninsulfonsäure, Naphthalinsulfonsäuren, Phenolsulfonsäure, Alkylarylsulfonate, Alkylsulfonate, Alkylsulfonate, Alkali- und Erdalkalisalze der Dibutylnaphthalinsulfonsäure, Lauryläthersulfat, Fettalkoholsulfate, fettsaure Alkali-, und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Napthalins bzw. der Napthalins bzw. der Napthalinsulfonsäuren mit Phenol und Formaldehyd, Polyoxyathylen-octylphenoläther, äthoxyliertes Isooctylphenyl, -Octylphenol, -Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglycolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Silikate, Talkum, Kaolin, Kalkstein, Kalk, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Die Wirkstoffe können auch mit anderen bekannten Fungiziden gemischt werden. In vielen Fällen erhält man dabei eine Vergrößerung des fungiziden Wirkungsspektrums; bei einer Anzahl dieser Fungizidmischungen treten synergistische Effekte auf, d.h. die fungizide Wirksamkeit des Kombinationsproduktes ist größer als die der addierten Wirksamkeit der Einzelkomponenten.

Fungizide, die mit den erfindungsgemäßen 4-Nitro-2-trichlormethylphenylsulfenamiden kombiniert werden können, sind beispielsweise:

Ferridimethyldithiocarbamat,
Zinkdimethyldithiocarbamat,
Manganäthylenbisdithiocarbamat,
Mangan-zink-äthylendiamin-bis-dithiocarbamat,
Zinkäthylenbisdithiocarbamat,
Tetramethylthiuramdisulfid,
3,5-Dimethyl-1,3,5-2H-tetrahydrothiadiazin-2-thion,
Ammoniak-Komplex von Zink-(N,N'-äthylen)-bis-dithiocarbamat und
N,N'-Polyäthylen-bis-(thiocarbamoyl)-disulfid,
Zink-(N,N'-propylen)-bis-dithiocarbamat,
Ammoniak-Komplex von Zink-(N,N'-propylen-bis-dithiocarbamat) und

N,N'-Polypropylen-bis-(thiocarbamoyl)-disulfid;
Dinitro-(1-methylheptyl)-phenyl-crotonat,
2-sec.-Butyl-4,6-dinitrophenyl-3,3,-dimethylacrylat,
2-sec.-Butyl-4,6-dinitrophenylisopropylcarbonat,
2,4,5-Trichlorphenol,
Pentachlorphenol,
Barium-Salz von Pentachlorphenol,
Pentachlorphenyl-acetat,
Pentachlorbenzylalkohol,
Di-(5-chloro-2-hydroxyphenyl)-methan,
Phenyl-(5-chloro-2-hydroxyphenyl)-methan,
N-Trichlormethylthio-tetrahydrophthalamid,
N-Trichlormethylthio-phthalimid,
N-Fluordichlormethylthio-phthalimid,
N-(1,1,2,2-Tetrachloräthylthio)-tetrahydrophthalimid,
2-Heptadecyl-2-imidazolinacetat,
2,4-Dichlor-6-(o-chloranilino)-s-triazin,
Diäthylphthalimidophosphorothioat,
5-Amino-1-[bis-(dimethylamino)-phosphinyl]-3-phenyl-1,2,4-triazol,
5-Äthoxy-3-trichlormethyl-1,2,4-thiadiazol,
2,3-Dicyano-1,4-dithioanthrachinon,
Chinoxalin-2,3-cycl.-trithiocarbonat,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
1-(1,2,4-Triazolyl-1')-[1-(4'-chlorphenoxy)]-3,3-dimethylbutan-2-on,
1-(1-Imidazoyl)-2-allyloxy-2-(2,4-dichlorphenyl)-äthan,
2-(O,O-Diäthyl-thionophosphoryl)-5-methyl-6-carbäthoxy-pyrazol-(1,5a)-pyrimidin,
Pyridin-2-thiol-1-oxid,
8-Hydroxychinolin bzw. dessen Kupfer-Salz,
5,5-Dimethyl-3-(3,5-dichlorphenyl)-2,4-dioxo-1,3-oxazolidin,
5-Methyl-5-vinyl-3-(3,5-dichlorphenyl)-2,4-dioxo-1,3-oxazolidin,
2-[Furyl-(2)]-benzimidazol,
Piperazin-1,4-diyl-bis-1-(2,2,2-Trichlor-äthyl)-formamid,
2-[Thiazolyl-(4)]-benzimidazol,
5-Butyl-2-dimethylamino-4-hydroxy-6-methyl-pyrimidin,
Bis-(p-chlorphenyl)-3-pyridinmethanol,
1,2-Bis-(3-äthoxycarbonyl-2-thioureido)-benzol,
1,2-Bis-(3-methoxycarbonyl-1-thioureido)-benzol,
Dodecylguanidinacetat,
3-[2-(3,5-Dimethyl-2-oxycyclohexyl)-2-hydroxyäthyl]-glutarimid,
Hexachlorbenzol,
N-Dichlorfluormethylthio-N',N'-dimethyl-N-phenyl-schwefelsäurediamid,
N-Dichlorfluormethylthio-N-methyl-N'-methyl-N'-phenyl-schwefelsäurediamid,
2,4,5,6-Tetrachlor-isophthalonitril,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,
p-Dimethylaminobenzol-diazonatriumsulfonat,
2-Chlor-1-nitro-propan,
Polychlornitrobenzole wie Pentachlornitrobenzol,
Methylisothiocyanat,
Triphenyl-zinn-acetat,
fungizide Antibiotika wie Griseofulvin oder
Kasugamycin,
Tetrafluordichloraceton,
1-Phenylthiosemicarbazid,
Aluminiumkomplex des N'-Hydroxy-N-cyclohexyl-diazeniumoxids,
Bordeauxmischung,
nickelhaltige Verbindungen und Schwefel,
Mercaptobenzthiazol,
Methyl-N-(2,6-dimethyl)-N-(2-furoyl)-alaninat,

Methyl-N-(2,6-dimethyl)-N-(2-methoxyacetyl)-alaninat,
2-Cyano-N-[(äthylamino)carbonyl]-2-(methoximino)-acetamid,
β-(4-Chlorphenoxy)-α-(1,1-dimethyl-1H-1,2,4-triazol-1-äthanol,
Benzisothioazolon.

Die Zumischung dieser Mittel zu den erfindungsgemäßen Fungiziden kann im Gewichtsverhältnis 1:10 bis 10:1 erfolgen. Sie können gegebenenfalls auch erst unmittelbar vor der Anwendung (Tankmischung) zugesetzt werden.

Beispiel 51.

Aspergillus niger

Die Wirkstoffe werden einer für das Wachstum des Pilzes Aspergillus niger optimal geeigneten Nährlösung in Mengen von 100, 50, 25 und 10 Gewichtsteilen pro Million Teile Nährlösung in 100 ml Glaskolben mit 0,3 mg Aspergillus-Pilzsporen beimpft. Die Kolben werden 120 Stunden lang auf 36°C erwärmt und anschließend das Ausmaß der Pilzentwicklung, das bevorzugt auf der Nährlösungsoberfläche erfolgt, beurteilt.

0 = kein Pilzwachstum, abgestuft bis 5 = umgehemmtes Pilzwachstum (Pilzdecke auf der Nährlösungsoberfläche geschlossen)

| Wirkstoff Beispiel Nr. | Wirkstoffmenge in der Nählösung ... Teile pro Million Teile Nährlösung 100 | 50 | 25 | 10 |
|---|---|---|---|---|
| 2 | 0 | 0 | 3 | 4 |
| 6 | 0 | 0 | 2 | 4 |
| 9 | 0 | 0 | 0 | 2 |
| 10 | 9 | 9 | 2 | 2 |
| 11 | 0 | 0 | 0 | 2 |
| 12 | 0 | 0 | 0 | 3 |
| 15 | 0 | 0 | 0 | 2 |
| 16 | 0 | 0 | 0 | 3 |
| 18 | 0 | 0 | 0 | 2 |
| 20 | 0 | 0 | 2 | 3 |
| 40 | 0 | 0 | 2 | 4 |
| 47 | 0 | 0 | 2 | 2 |
| 49 | 0 | 2 | 2 | 3 |
| Vergleichsmittel | | | | |
| 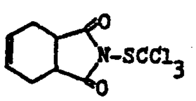 | 1 | 1 | 3 | 4 |
| (bekannt) | | | | |
| 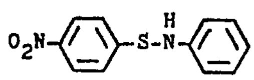 | 5 | 5 | 5 | 5 |
| 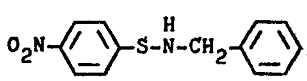 | 5 | 5 | 5 | 5 |
| 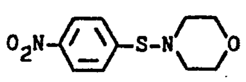 | 5 | 5 | 5 | 5 |
| Kontrolle (unbehandelt) | 5 | | | |

Beispiel 52.

Plasmopara viticola an Reben

Blätter und Topfreben der Sorte Müller-Thurgau werden mit wäßrigen Dispersionen, die 80% (Gew.%) des zu prüfenden Wirkstoffs und 20% Natriumligninsulfonat in der Trockensubstanz enthalten, besprüht. Es werden 0,05- und 0,25%-ige Spritzbrühen (bezogen auf die Trockensubstanz) verwendet. Nach dem Antrocknen des Spritzbelages werden die Blätter mit einer Zoosporenaufschwemmung von Plasmopara viticola infiziert. Die Pflanzen kommen dann zuerst für 16 Stunden in eine wasserdampfgesättigte (feuchte) Kammer bei 20°C und anschließend 8 Tage in ein Gewächshaus mit Temperaturen zwischen 20 und 30°C. Nach dieser Zeit werden die Pflanzen zur Beschleunigung und Verstärkung des Sporangienträgerausbruches abermals während 16 Stunden in der feuchten Kammer aufgestellt. Dann erfolgt die Beurteilung des Blattbefalls auf den Blattunterseiten. Als Vergleich dienen unbehandelte infizierte Kontrollpflanzen.

| Wirkstoff Beispiel Nr. | Blattbefall nach Spritzung mit ... %-iger Spritzbrühe 0,05 | 0,025 |
|---|---|---|
| 8 | 0 | 0 |
| 12 | 0 | 2 |
| 15 | 0 | 0 |
| 16 | 0 | 0 |
| 18 | 0 | 2 |
| 27 | 0 | 0 |
| 39 | 0 | 0 |
| Vergleichsmittel 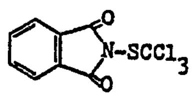 | 2 | 3 |
| Kontrolle (unbehandelt) | 5 | |

0 = kein Befall, abgestuft bis 5 = Totalbefall

Beispiel 53.

Bakterizide Wirksamkeit gegenüber Staphylococcus aureus

Die Abtötungswerte gegenüber Staphylococcus aureus wurden wie folgt ermittelt: Zu je 5 ml einer Verdünnung der Wirkstoffe im Verhältnis 1:5000 in Wasser wurden 5 ml doppelt-konzentrierter Nährbouillon in sterilen Reagenzgläsern gegeben und vermischt. Durch Zugabe von einem Tropfen einer im Gewichtsverhältnis 1:10 mit Wasser verdünnten 16 Stunden alten Bouillon-Kultur des Bakteriums Staphylococcus aureus wurden dann die Reagenzgläser beimpft und 24 Stunden lang auf 37°C erwärmt. Nach dieser Zeit wurden die Proben aus den Reagenzgläsern auf Bakteriennährböden übertragen und diese ebenfalls 24 Stunden lang auf 37°C erwärmt. Bei den folgenden Wirkstoffen erfolgte nach dem Übertragen auf den Nährboden keine Bakterienentwicklung mehr:

Wirkstoffe Nr.: 6, 10, 12, 16, 18 und 26

Beispiel 54.

Man vermischt 90 Gewichtsteile der Verbindung 1 mit 10 Gewichtsteilen N-Methyl-$\alpha$-pyrrolidon und erhält eine Lösung, die zur Anwendung in Form kleinster Tropfen geeignet ist.

Beispiel 55.

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an salz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 10 000 Gewichtsteilen Wasser erhält man eine wäßrige Dispersion, die 0,02 Gew.% des Wirkstoffs enthält.

Beispiel 56.

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Beispiel 57.

20 Gewichtsteile der Verbindung 1 werden in einer Mischung gelöst, die aus 25 Gewichtsteilen Cyclohexanol, 65 Gewichtsteilen einer Mineralölfraktion von Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Beispiel 58.

20 Gewichtsteile des Wirkstoffs 3 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 59.

3 Gewichtsteile der Verbindung 3 werden mit 97 Gewichtsteilen feinteiligem Kaolin innig vermischt. Man erhält auf diese Weise ein Stäubemittel, das 3 Gew.% des Wirkstoffs enthält.

Beispiel 60.

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhählt auf diese Weise eine Aufbereitung des Wirkstoffs mit guter Haftfähigkeit.

Beispiel 61.

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

Beispiel 62.

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche für die Vertragsstaaten: BE, CH, DE, FR, GB, IT, LU, NL, SE**

1. 4-Nitro-2-trichlormethylphenylsulfenamid der Formel

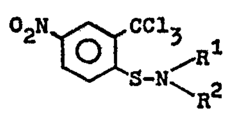

in der $R^1$ und $R^2$ gleich oder voneinander verschieden sind und Wasserstoff, einen gegebenenfalls durch Alkoxy, Hydroxy, Trialkoxysilyl substituierten Alkylrest mit 1 bis 18 Kohlenstoffatomen, einen Alkenylrest mit 3 bis 5 Kohlenstoffatomen, Cycloalkylrest mit 5 bis 8 Kohlenstoffatomen, einen gegebenenfalls durch Halogen substituierten Aralkylrest mit 7 bis 9 Kohlenstoffatomen, einen gegebenenfalls durch Alkyl, Halogen, Nitro, Halogenalkyl substituierten Phenylrest bedeuten, $R^1$ ferner einen 1 bis 2 Heteroatome enthaltenden 5- bis 6-gliedrigen ungesättigten Methylenring mit Stickstoff oder Schwefel als Heteroatomen oder dessen Benzhomologe bedeuten oder $R^1$ und $R^2$ zusammen mit dem Stickstoffatom, dessen Substituenten sie sind, einen 5- bis 7-gliedrigen gegebenenfalls ungesättigten Methylenring bedeuten, der gegebenenfalls durch Alkyl, Phenyl, Nitro substituiert ist und gegebenenfalls im Methylring noch Stickstoff oder Sauerstoff enthalten kann und $R^1$ ferner den Rest 4-Nitro-2-trichlormethylphenylsulfenyl bedeutet.

2. Fungizid, enthaltend ein 4-Nitro-2-trichlormethylphenylsulfamid gemäß Anspruch 1.

3. Fungizid, enthaltend einen festen oder flüssigen Trägerstoff und ein 4-Nitro-2-trichlormethylphenylsulfenamid gemäß Anspruch 1.

4. Verfahren zur Bekämpfung von Pilzen, dadurch gekennzeichnet, daß man die Pilze oder die vor Pilzbefall zu schützenden Gegenstände behandelt mit einem 4-Nitro-2-trichlormethylphenylsulfenamid gemäß Anspruch 1.

5. 4-Nitro-2-trichlormethylphenylsulfenamid, ausgewählt aus der Gruppe, bestehend aus N-(Octyl-(1)-)-4-nitro-2-trichlormethylphenylsulfenamid, N-(n-Propyl-(1)-)-4-nitro-2-trichlormethylphenylsulfenamid.

6. Fungizid, enthaltend ein 4-Nitro-2-trichlormethylphenylsulfenamid ausgewählt aus der Gruppe N-(Octyl-(1)-)-4-nitro-2-trichlormethylphenylsulfenamid, N-(n-Propyl-(1)-)-4-Nitro-2-trichlormethylphenylsulfenamid.

**Claims for the Contracting States: BE, CH, DE, FR, GB, IT, LU, NL, SE**

1. A 4-nitro-2-trichloromethylphenylsulfenamide of the formula

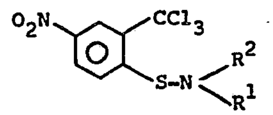

where $R^1$ and $R^2$ are identical or different and each denotes hydrogen, alkyl of from 1 to 18 carbon atoms optionally substituted by alkoxy, hydroxy or trialkoxysilyl, alkenyl of from 3 to 5 carbon atoms, cycloalkyl of from 5 to 8 carbon atoms, aralkyl of from 7 to 9 carbon atoms optionally substituted by halogen, phenyl optionally substituted by alkyl, halogen, nitro or haloalkyl, $R^1$ also denotes a 5- or 6-membered unsaturated methylene ring containing 1 or 2 nitrogen or sulfur atoms as hetero atoms, or its benz homologs, or $R^1$ and $R^2$, together with the nitrogen atom whose substituents they are, denote a 5- to 7-membered saturated or unsaturated methylene ring which is optionally substituted by alkyl, phenyl or nitro and may or may not contain nitrogen or oxygen atoms in the methylene ring, and $R^1$ further denotes 4-nitro-2-trichloromethylphenylsulfenyl.

2. A fungicide containing a 4-nitro-2-trichloromethylphenylsulfenamide as claimed in claim 1.

3. A fungicide containing a solid or liquid carrier and a 4-nitro-2-trichloromethylphenylsulfenamide as claimed in claim 1.

4. A process for combating fungi, characterized in that the fungi or the objects to be protected against fungus attack are treated with a 4-nitro-2-trichloromethylphenylsulfenamide as claimed in claim 1.

5. A 4-nitro-2-trichloromethylphenylsulfenamide selected from the group consisting of N-(octyl-(1))-4-nitro-2-trichloromethylphenylsulfenamide and N-(n-propyl-(1))-4-nitro-2-trichloromethylphenylsulfenamide.

6. A fungicide containing a 4-nitro-2-trichloromethylphenylsulfenamide selected from the group consisting of N-(octyl-(1))-4-nitro-2-trichloromethylphenylsulfenamide and N-(n-propyl-(1))-4-nitro-2-trichloromethylphenylsulfenamide.

**Revendications pour les Etats contractants: BE, CH, DE, FR, GB, IT, LU, NL, SE**

1. 4-Nitro-2-trichlorométhylphényl-sulfenamide de la formule générale

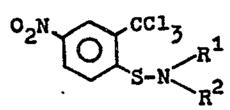

dans laquelle $R^1$ et $R^2$, qui peuvent être identiques ou différents, représentent chacun un atome d'hydrogène, un radical alcoyle en $C_1$ à $C_{18}$, éventuellement substitué par des groupes alcoxy, hydroxy ou trialcoxy-silyle, un radical alcényle en $C_3$ à $C_5$, un groupe cyclo-alcoyle en $C_5$ à $C_8$, un groupe aralcoyle en $C_7$ à $C_9$, éventuellement halosubstitué, un groupe phényle éventuellement substitué par des groupes alcoyle, halogène, nitro ou halo-alcoyle, $R^1$ pouvant en outre désigner un noyau méthylénique non saturé à 5 ou 6 chaînons, comprenant 1 ou 2 hétéro-atomes choisis parmi l'azote et le soufre, ou ses homologues benzyliques ou $R^1$ et $R^2$ pouvant former avec l'atome d'azote, dont ils sont les substituants, un noyau méthylénique saturé ou non avec 5 à 7 chaînons, éventuellement substitué par des groupes alcoyle, phényle ou nitro, pouvant comporter d'autres hétéro-atomes choisis parmi l'azote et l'oxygène, $R^1$ pouvant de plus désigner le groupe 4-nitro-2-trichlorométhylphényl-sulfényle.

2. Composition fongicide, contenant un 4-nitro-2-trichlorométhylphényl-sulfénamide suivant la revendication 1.

3. Composition fongicide, composée d'un véhicule solide ou liquide et d'un 4-nitro-2-trichlorométhylphényl-sulfénamide suivant la revendication 1.

4. Procédé de lutte contre les champignons, caractérisé en ce que l'on applique sur les champignons ou sur les objets à protéger des champignons un 4-nitro-2-trichlorométhylphényl-sulfénamide suivant la revendication 1.

5. 4-Nitro-2-trichlorométhylphényl-sulfénamide suivant la revendication 1, choisi parmi le N-[octyl-(1)]-4-nitro-2-trichlorométhylphényl)-sulfénamide et le N-[n-propyl-(1)]-4-nitro-2-trichlorométhylphényl-sulfénamide.

6. Composition fongicide, contenant un 4-nitro-2-trichlorométhylphényl-sulfénamide choisi parmi le N-[octyl-(1)]-4-nitro-2-trichlorométhylphényl-sulfénamide et le N-[n-propyl-(1)]-4-nitro-2-trichlorométhylphényl-sulfénamide.

**Patentansprüche für den Vertragsstaat AT**

1. Fungizid, enthaltend ein 4-Nitro-2-trichlormethylphenyl-sulfamid der Formel

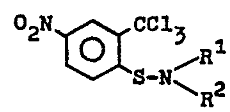

in der $R^1$ und $R^2$ gleich oder voneinander verschieden sind und Wasserstoff, einen gegebenenfalls durch Alkoxy, Hydroxy, Trialkoxysilyl substituierten Alkylrest mit 1 bis 18 Kohlenstoffatomen, einen Alkenylrest mit 3 bis 5 Kohlenstoffatomen, Cycloalkylrest mit 5 bis 8 Kohlenstoffatomen, einen gegebenenfalls durch Halogen substituierten Aralkylrest mit 7 bis 9 Kohlenstoffatomen, einen gegebenenfalls durch Alkyl, Halogen, Nitro, Halogenalkyl substituierten Phenylrest bedeuten, $R^1$ ferner einen 1 bis 2 Heteroatome enthaltenden 5- bis 6-gliedrigen ungesättigten Methylenring mit Stickstoff oder Schwefel als Heteroatomen oder dessen Benzhomologe bedeuten oder $R^1$ und $R^2$ zusammen mit dem Stickstoffatom, dessen Substituenten sie sind, einen 5- bis 7-gliedrigen gegebenenfalls ungesättigten Methylenring bedeuten, der gegebenenfalls durch Alkyl, Phenyl, Nitro substituiert ist und gegebenenfalls im Methylring noch Stickstoff oder Sauerstoff enthalten kann und $R^1$ ferner den Rest 4-Nitro-2-trichlormethylphenylsulfenyl bedeutet.

2. Fungizid, enthaltend einen festen oder flüssigen Trägerstoff und ein 4-Nitro-2-trichlormethylphenylsulfenamid wie in Anspruch 1 definiert.

3. Verfahren zur Bekämpfung von Pilzen, dadurch gekennzeichnet, daß man die Pilze oder die vor Pilzbefall zu schützenden Gegenstände behandelt mit einem 4-Nitro-2-trichlormethylphenylsulfenamid wie in Anspruch 1 definiert.

4. Fungizid, enthaltend ein 4-Nitro-2-trichlormethylphenylsulfenamid ausgewählt aus der Gruppe N-(Octyl-(1)-)-4-nitro-2-trichlormethylphenylsulfenamid, N-(n-Propyl-(1)-)-4-Nitro-2-trichlormethylphenylsulfenamid.

**Claims for the Contracting State: AT**

1. A fungicide containing a 4-nitro-2-trichloromethylphenylsulfenamide of the formula

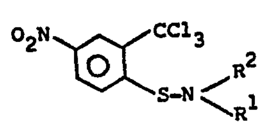

where $R^1$ and $R^2$ are identical or different and each denotes hydrogen, alkyl of from 1 to 18 carbon atoms optionally substituted by alkoxy, hydroxy or trialkoxysilyl, alkenyl of from 3 to 5 carbon atoms, cycloalkyl of from 5 to 8 carbon atoms, aralkyl of from 7 to 9 carbon atoms optionally substituted by halogen, phenyl optionally substituted by alkyl, halogen, nitro or haloalkyl, $R^1$ also denotes a 5- or 6-membered unsaturated methylene ring containing 1 or 2 nitrogen or sulfur atoms as hetero atoms, or its benz homologs, or $R^1$ and $R^2$, together with the nitrogen atom whose substituents they are, denote a 5- to 7-membered saturated or unsaturated methylene ring which is optionally substituted by alkyl, phenyl or nitro and may or may not contain nitrogen or oxygen atoms in the methylene ring, and $R^1$ further denotes 4-nitro-2-trichloromethylphenylsulfenyl.

2. A fungicide containing a solid or liquid carrier and a 4-nitro-2-trichloromethylphenylsulfenamide as defined in claim 1.

3. A process for combating fungi, characterized in that the fungi or the objects to be protected against fungus attack are treated with a 4-nitro-2-trichloromethylphenylsulfenamide as defined in claim 1.

4. A fungicide containing a 4-nitro-2-trichloromethylphenylsulfenamide selected from the group consisting of N-(octyl-(1))-4-nitro-2-trichloromethylphenylsulfenamide and N-(n-propyl-(1))-4-nitro-2-trichloromethylphenylsulfenamide.

**Revendications pour l'Etat contractant: AT**

1. Composition fongicide, contenant un 4-nitro-2-trichlorométhylphényl-sulfénamide de la formule

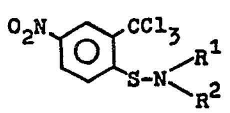

dans laquelle $R^1$ et $R^2$, qui peuvent être identiques ou différents, représentent chacun un atome d'hydrogène, un radical alcoyle en $C_1$ à $C_{18}$, éventuellement substitué par des groupes alcoxy, hydroxy ou trialcoxy-silyl, un radical alcényle en $C_3$ à $C_5$, un groupe cyclo-alcoyle en $C_5$ à $C_8$, un groupe aralcoyle en $C_7$ à $C_9$, éventuellement halosubstitué, un groupe phényle éventuellement substitué par des groupes alcoyle, halogène, nitro ou halo-alcoyle, $R^1$ pouvant en outre désigner un noyau méthylénique non saturé à 5 ou 6 chaînons, comprenant 1 ou 2 hétéro-atomes choisis parmi l'azote et le soufre, ou ses homologues benzyliques ou $R^1$ et $R^2$ pouvant former avec l'atome d'azote, dont ils sont les substituants, un noyau méthylénique saturé ou non avec 5 à 7 chaînons, éventuellement substitué par des groupes alcoyle, phényle ou nitro, pouvant comporter d'autres hétéro-atomes choisis parmi l'azote et l'oxygène, $R^1$ pouvant de plus désigner le groupe 4-nitro-2-trichlorométhylphényl-sulfényle.

2. Composition fongicide, contenant un véhicule solide ou liquide et un 4-nitro-2-trichlorométhylphényl-sulfénamide tel que défini dans la revendication 1.

3. Procédé de lutte contre les champignons, caractérisé en ce que l'on traite les champignons ou les objets à protéger des champignons avec un 4-nitro-2-trichlorométhylphényl-sulfénamide tel que défini dans la revendication 1.

4. Composition fongicide, contenant un 4-nitro-2-trichlorométhylphényl-sulfenamide choisi parmi le N-[octyl-(1)]-4-nitro-2-trichlorométhylphényl-sulfénamide et le N-[n-propyl-(1)]-4-nitro-2-trichlorométhylphényl-sulfénamide.